(19) Europäisches Patentamt
European Patent Office
Office européen des brevets

(11) **EP 0 826 417 A1**

(12) **EUROPÄISCHE PATENTANMELDUNG**

(43) Veröffentlichungstag:
04.03.1998 Patentblatt 1998/10

(51) Int. Cl.$^6$: **B01J 23/18**, C01B 7/04,
C07F 9/94

(21) Anmeldenummer: 97114611.3

(22) Anmeldetag: 22.08.1997

(84) Benannte Vertragsstaaten:
AT BE CH DE DK ES FI FR GB GR IE IT LI LU MC
NL PT SE
Benannte Erstreckungsstaaten:
AL LT LV RO SI

(30) Priorität: 23.08.1996 DE 19634192

(71) Anmelder:
BASF AKTIENGESELLSCHAFT
67056 Ludwigshafen (DE)

(72) Erfinder:
• Hagemeyer, Alfred, Dr.
48431 Rheine (DE)
• Püttner, Andreas, Dr.
60431 Frankfurt (DE)
• Trömel, Martin, Prof. Dr.
61184 Karben (DE)

(74) Vertreter:
Kinzebach, Werner, Dr. et al
Patent Attorneys,
Reitstötter, Kinzebach & Partner,
Sternwartstrasse 4
81679 München (DE)

(54) **Bismut-haltige Katalysatoren**

(57) Die vorliegende Erfindung betrifft eine Katalysatorzusammensetzung, dadurch gekennzeichnet, daß deren Aktivphase wenigstens eine Bismutoxidverbindung enthält, worin Bismut wenigstens teilweise in der Oxidationsstufe +5 vorliegt, wobei die Bismutoxidverbindung außerdem wenigstens eine die Oxidationsstufe +5 stabilisierende, basische Metallkomponente enthält, sowie deren Verwendung in heterogen katalysierten Oxidations- und Dehydrierreaktionen, wie insbesondere der Chlorherstellung aus Chlorwasserstoff.

## Beschreibung

Die Erfindung betrifft Bismutoxidverbindungen enthaltende Katalysatoren, deren Verwendung in heterogen katalysierten Oxidations- und Dehydrierreaktionen, Verfahren zur Herstellung von Chlor unter Verwendung dieser Katalysatoren sowie neuartige Barium-Bismutoxidphasen und Verfahren zu deren Herstellung.

Bei vielen chemischen Umsetzungen, wie z.B. der Phosgenierung von Diaminen zur Herstellung von Isocyanaten für die Polyurethansynthese oder bei der Vinylchlorid-Produktion, fällt HCl in großen Mengen als Nebenprodukt an. Für die Verwertung der anfallenden HCl gibt es verschiedene Möglichkeiten: Vermarktung, Weiterverarbeitung z.B. in der Oxichlorierung, Entsorgung durch Neutralisation, Einsatz zur Herstellung anorganischer Chloride oder Recycling zu Chlor. Da die Entwicklung des Marktes für HCl schwierig zu beurteilen ist und angesichts einer stetig steigenden HCl-Menge aus Produktionsprozessen, besteht ein großer Bedarf an HCl-Recyclingverfahren, bei denen Chlor als Wertstoff wiedergewonnen wird.

Ein etabliertes Recylingverfahren ist z.B. die Elektrolyse von HCl oder $CuCl_2$ nach dem Westvaco-Prozess, wie er von J. Gordon, Chem. Eng. 1953, S. 187; und Anon, Chem. Eng. 1960, S. 63, beschrieben wird. Nachteilig bei diesem Verfahren sind jedoch die hohen Energiekosten.

Aus dem Stand der Technik sind bereits seit langem verschiedene Verfahren zur Chlorherstellung durch HCl-Oxidation bekannt. Das älteste ist das 1868 von Deacon entwickelte Verfahren zur direkten und kontinuierlichen Umsetzung von HCl mit Luft oder Sauerstoff an einem Kupferchloridkatalysator (vgl. z.B. die Übersichtsartikel von Kepinski, J., Tilly, J., Katucki, K. in Przem. Chem. 57(1) (1978), S. 14-17 und Kepinski, J., Kalucki, K. in Szczecin. Tow. Nauk., Wydz. Nauk. Mat. Tech. 9, 1973, S. 37-49). Die Reaktion ist gleichgewichtslimitiert, so daß maximal 75% Umsatz erreicht werden. Der Produktstrom enthält somit neben $Cl_2$ noch HCl, $H_2O$ und Luft/Sauerstoff. Dies macht eine nachfolgende, aufwendige Aufarbeitung des enthaltenen $Cl_2$ erforderlich, wobei aufgrund der im Produktgas enthaltenen wäßrigen Salzsäure gravierende Korrosionsprobleme auftreten.

Ein modifiziertes, großtechnisches Verfahren ist der Shell-Deacon-Prozeß, bei dem HCl heterogenkatalytisch an einem geträgerten $CuCl_2/KCl/LaCl_3$-Katalysator mit Luft in einem Wirbelschichtreaktor mit etwa 77% Ausbeute zu Chlor oxidiert wird. Dieses Verfahren wird z.B. von J.Th. Quant et al. in The Chemical Engineer, July/August 1963, S. 224, beschrieben.

Ein weiteres Verfahren, das früher technisch verwendet wurde, ist der Weldon-Prozeß, bei dem Braunstein zur Oxidation von HCl eingesetzt wird. Die Chlorausbeute betrug jedoch nur etwa 30%, da die Hälfte des eingesetzten HCl bei der Rückführung des $MnCl_2$ zu $MnO_2$ unter Verwendung von $Ca(OH)_2$ und $O_2$ in Form von $CaCl_2$ wieder verlorenging.

Beim Kel-Chlor-Prozeß von Kellog wird HCl bei erhöhtem Druck und erhöhter Temperatur mit Nitrosylschwefelsäure zu Chlor umgesetzt. Auch hier führt das Problem der Korrosion zum Einsatz teurer Werkstoffe und somit zu hohen Investitionskosten.

Die FR 14 97 776 beschreibt eine Variante des Deacon-Verfahrens, wobei der Katalysator in Form einer Carborundum-$CuCl_2$-KCl-Salzschmelze ("supported liquid phase") verwendet wird. Bei den auftretenden Reaktionstemperaturen von etwa 400°C kommt es bei diesem Verfahren jedoch zu einem starken Austrag der flüchtigen Kupferchloride und somit zu Katalysatorverlusten und Verunreinigungen auf den Reaktor folgenden Leitungsteile.

Eine weitere Variante des Deacon-Verfahrens stellt das Mitsui-Toatsu-Verfahren dar, bei dem HCl in der Wirbelschicht an $Cr_2O_3/SiO_2$-Katalysatoren oxidiert wird, wobei der HCl-Umsatz 75 bis 80% beträgt. Dieses Verfahren ist in EP 0 184 413, EP 0 277 332, EP 0 331 465, EP 0 465 243 und JP 62 254 846 beschrieben. Nachteilig bei diesem Verfahren ist die hohe Toxizität des in den verwendeten $Cr_2O_3$-Katalysatoren enthaltenen Chroms.

Bei der Chlorherstellung durch HCl-Oxidation kann man zwischen Verfahren mit stationärer und instationärer Reaktionsführung unterscheiden. Bei der konventionellen, stationären Reaktionsführung wird die HCl-haltige Einspeisung (Feed) zusammen mit einem sauerstoffhaltigem Gas und eventuell weiteren Verdünnungsgasen zeitlich kontinuierlich mit dem Katalysatorbett in Kontakt gebracht, wobei das enthaltene HCl teilweise zu $Cl_2$ und $H_2O$ oxidiert wird, und die Reaktionsprodukte zusammen mit nicht umgesetztem HCl, $O_2$ und Trägergas den Reaktor kontinuierlich verlassen. Da die Reaktion gleichgewichtslimitiert ist, ist nur ein Teilumsatz möglich.

Bei den aus dem Stand der Technik bekannten instationären Verfahren erfolgt die HCl-Oxidation in zwei Teilschritten, wobei der Katalysator als Stoffspeicher, genauer als Chlorspeicher, fungiert. Im Beladeschritt wird der Katalysator mit HCl chloriert und seine oxidische Aktivkomponentenphase in eine Chloridphase und Wasser umgewandelt. Nach einer kurzen Spülphase mit Inertgas strömt im zweiten Schritt ein sauerstoffhaltiges Gas über den beladenen Katalysator. Dabei wird Chlor freigesetzt und die oxidische Phase zurückgebildet.

Die DE 40 04 454 beschreibt ein Verfahren zur Gewinnung von Chlor durch Oxidation von HCl über zwei Verfahrensstufen unter Verwendung eines Transportkatalysators. Dabei wird in der ersten Stufe ein HCl-Gasstrom durch ein Fließbett aus Kupferoxiden und NaCl, die auf einem geeigneten Träger aufgebracht sind, geführt und durch Reaktion ein komplexes Chlorid gebildet. Nach Abführung des Fließbetts zur Dechlorierung in einen zweiten Reaktor erfolgt die Rückführung des oxidierten Transportkatalysators unter Eindüsung

von $O_2$ und $N_2$. Die US 49 59 202 und EP 04 74 763 beschreiben ebenfalls ein in zwei Reaktoren durchgeführtes instationäres Verfahren, bei dem die HCl-Beladung des Katalysators ebenso wie die Dechlorierung im Wirbelbett erfolgt.

Die WO 91/06505 und die US 51 54 911 beschreiben einen modifizierten Deacon-Prozeß mit instationärer Reaktionsführung unter Verwendung eines Katalysators, der

a) ein Übergangsmetalloxid, ausgewählt unter $MnO_2$, $Co_2O_3$, $Co_3O_4$, $Cr_2O_3$, $NiO$, $Ni_2O_3$, $Mo_2O_3$, $CuO$ und deren Kombinationen,
b) ein Alkalimetallchlorid, ausgewählt unter LiCl, NaCl, KCl und deren Kombinationen,
c) ein Promotor, ausgewählt unter $LaCl_3$, $PrCl_3$, $Pr_2O_3$ und deren Kombinationen,

umfaßt.

Das Verfahren umfaßt einen Chlorierungs- und einen Oxidationsschritt und erfolgt in einem Wirbelbett- oder Festbettreaktor, wobei gegebenenfalls ein Austausch des Katalysatorbetts zwischen den Reaktionszonen erfolgen kann. Ein ähnliches Verfahren unter Verwendung eines Festbettreaktors wird in der EP 0 500 728 beschrieben.

Die DE 43 36 404 beschreibt ebenfalls einen modifizierten Deacon-Prozeß mit instationärer Reaktionsführung. Es wird vorgeschlagen, das eingesetzte HCl-Gas mit einem Molekularsieb zu trocknen, um das während der Beladephase entstehende Reaktionswasser zu binden. Zur Oxidation soll reiner Sauerstoff bei Drükken zwischen 1,0 und 50 bar und Temperaturen zwischen 100 und 500°C verwendet werden. Als Katalysatoren werden Mangan- und Vanadium-Oxide vorgeschlagen. Als problematisch bei diesem Verfahren sind die hohe Flüchtigkeit von Vanadylchloriden bzw. die zu hohe Aktivität von $MnO_2$ anzusehen, so daß mit Chlorbildung schon während der Beladephase zu rechnen ist. Ferner ist die Korrosionsbeständigkeit der vorgeschlagenen Molsiebzeolithe fraglich. Aufgrund der hohen Azidität von Zeolithen muß von beträchtlicher HCl-Adsorption am Molekularsieb ausgegangen werden, die mit ebenfalls absorbiertem Wasser Salzsäure bildet und den Träger angreift.

H.Y. Pan, R.G. Minett, S.W. Benson und T.T. Tsotsis beschreiben in Ind. Eng. Chem. Res. 33 (1994), S. 2996-3003 ein Reaktorkonzept unter Kopplung von zwei alternierend betriebenen Wirbelschichtreaktoren, wobei als Katalysator ein geträgertes $CuCl_2$-NaCl-System verwendet wird.

Die bekannten Verfahren weisen somit spezifische Nachteile auf, die vor allem aus einer unvollständigen Umsetzung des eingesetzten Chlorwasserstoffs und den damit auftretenden Korrosionsproblemen oder der Notwendigkeit zu apparativ aufwendigen und somit kostspieligen Produktionsverfahren resultieren.

Der vorliegenden Erfindung liegt deshalb die Aufgabe zugrunde, geeignete Katalysatoren bereitzustellen, mit deren Hilfe aus dem Stand der Technik bekannte Probleme bei der HCl-Oxidation behoben werden können. Insbesondere sollten die Katalysatoren die $Cl_2$-Herstellung mit einer verbesserten Raum-Zeit-Ausbeute ermöglichen.

Diese Aufgabe wird gelöst durch Bereitstellung von Katalysatoren, deren Aktivphase eine oder mehrere Bismutoxidverbindungen enthält, die Sauerstoffspeicherfunktion besitzen.

Gegenstand der vorliegenden Erfindung sind somit Katalysatorzusammensetzungen, die dadurch gekennzeichnet sind, daß deren Aktivphase wenigstens eine Bismutoxidverbindung enthält, worin Bismut wenigstens teilweise in der Oxidationsstufe +5 vorliegt, wobei die Bismutoxidverbindung außerdem wenigstens eine die Oxidationsstufe +5 stabilisierende, basische Metallkomponente enthält.

Ein wesentliches Merkmal der erfindungsgemäßen Katalysatoren ist, daR Bismutoxidverbindinngen verwendet werden, bei denen Bi wenigstens teilweise in der Oxidationsstufe +5 vorliegt und sie daher über ein hohes Oxidationspotential verfügen, das eine hohe Katalysatoraktivität bedingt.

Vorzugsweise liegt der Anteil von Bi +5, bezogen auf den Gesamtbismutgehalt, bei etwa 5 bis 90 Gew.-%, vorzugsweise etwa 20 bis 80 Gew.-%.

Zur Stabilisierung der hohen Oxidationsstufe +5 ist eine stark basische Umgebung erforderlich. Dafür kommen Alkali- und Erdalkalimetalle sowie die Seltenerdmetalle in Frage. In einer bevorzugten Ausführungsform enthält der Katalysator eines oder mehrere der Elemente Li, Na, K, Sr, Ba, Cs, Y und La. Je nach Basizität dieser Komponenten ist ein unterschiedlicher Anteil an basischen Zuschlägen notwendig.

Besonders bevorzugte Katalysatoren sind dadurch gekennzeichnet, daß die Aktivphase mindestens eine Verbindung der allgemeinen Formel

$$KBi_yO_z$$

worin

y für einen Wert von 0,5 bis 2 und
z für einen Wert von 1,25 bis 5,5 steht,

wobei insbesondere y/z im Bereich von etwa 0,4 bis etwa 0,36 liegt;
oder der allgemeinen Formel:

$$BaBi_yO_z$$

worin

y für einen Wert von 0,17 bis 3 und
z für einen Wert von 1,43 bis 5,6 steht,

wobei insbesondere y/z im Bereich von etwa 0,12 bis etwa 0,54 liegt,
enthält,
wobei Bi jeweils wenigstens teilweise in der Oxidationsstufe +5 vorliegt.

Die Aktivphase des Katalysators kann gegebenenfalls Promotoren, insbesondere aus der 1., 2. und/oder 4. Hauptgruppe sowie der 4. und/oder 6. Nebengruppe des Periodensystems enthalten, die die Katalysatorwirkung verstärken. Beispiele dafür sind Si, Ge, Ti und/oder Cr.

Die Herstellung der erfindungsgemäßen Bismutoxid-Katalysatoren erfolgt in an sich bekannter Weise, wobei sowohl naßchemische Methoden, wie Fällen oder Tränken, als auch Festkörperreaktionen, wie z.B. Kalzinieren, eingesetzt werden können. Als Edukte können z.B. Carbonate, Hydroxide, Oxide, Peroxide, Nitrate oder Acetate verwendet werden. Die auf diese Weise hergestellten Feststoffe werden dann in an sich bekannter Weise zu den katalytisch aktiven Massen verarbeitet. Verfahren zur Katalysatorherstellung sind z.B. in Ullmanns Enzyklopädie der technischen Chemie, 3. Aufl., Bd. 9, S. 271 ff. (1957) beschrieben.

Nach einer speziellen Ausführungsform werden Barium-Bismutoxidverbindungen enthaltende Katalysatoren bereitgestellt. Die Herstellung von einigen Barium-Bismutoxidverbindungen durch Umsetzung stöchiometricher Mischungen aus Bariumcarbonat und/oder -nitrat und Bismutoxid wird von M. Itho et al., Solid State Ionics 49, S. 57-62 (1991) beschrieben.

Folgende Verbindungen werden von Itoh beschrieben:

$BaBiO_3$ ($\triangleq Ba_1Bi_1O_3$)
$Ba_{1,05}Bi_{0,95}O_{2,998}$ ($\triangleq Ba_1Bi_{0,905}O_{2,855}$)
$Ba_{1,10}Bi_{0,90}O_{2,994}$ ($\triangleq Ba_1Bi_{0,818}O_{2,722}$)
$Ba_{1,15}Bi_{0,85}O_{2,989}$ ($\triangleq Ba_1Bi_{0,739}O_{2,599}$)
$Ba_{1,20}Bi_{0,80}O_{2,984}$ ($\triangleq Ba_1Bi_{0,667}O_{2,487}$)
$Ba_{1,25}Bi_{0,75}O_{2,972}$ ($\triangleq Ba_1Bi_{0,600}O_{2,378}$)
$Ba_{1,30}Bi_{0,70}O_{2,970}$ ($\triangleq Ba_1Bi_{0,538}O_{2,285}$)
$Ba_{1,33}Bi_{0,67}O_{2,950}$ ($\triangleq Ba_1Bi_{0,504}O_{2,218}$)
$Ba_{1,40}Bi_{0,60}O_{2,880}$ ($\triangleq Ba_1Bi_{0,429}O_{2,057}$)
$Ba_{1,425}Bi_{0,575}O_{2,862}$ ($\triangleq Ba_1Bi_{0,404}O_{2,008}$)
$Ba_{1,45}Bi_{0,55}O_{2,825}$ ($\triangleq Ba_1Bi_{0,379}O_{1,948}$)
$Ba_{1,475}Bi_{0,525}O_{2,788}$ ($\triangleq Ba_1Bi_{0,355}O_{1,890}$) und
$Ba_{1,50}Bi_{0,50}O_{2,753}$ ($\triangleq Ba_1Bi_{0,333}O_{1,835}$)

Ein allgemeines Verfahren zur Herstellung der erfindungsgemäßen Barium-Bismutoxid-Katalysatoren umfaßt die Umsetzung einer oder mehrerer Bariumverbindungen, insbesondere Bariumnitrat, -oxid, -peroxid, -hydroxid, oder -carbonat mit einer oder mehreren Bismutverbindungen, insbesondere Bismutoxid, -nitrat, -carbonat oder -hydroxid, wobei die Reaktion naßchemisch oder als Festkörperreaktion erfolgen kann.

Insbesondere bevorzugt zur Herstellung der erfindungsgemäßen Barium-Bismutoxid-Katalysatoren ist die Festkörperreaktion unter Verwendung von Bariumcarbonat und/oder Bariumperoxid und von Bismutoxid.

Dazu werden z.B. $BaCO_3$ und $Bi_2O_3$ in stöchiometrischen Mengen in Aceton homogenisiert und bei einer Temperatur von 700° bis 900°C, bevorzugt etwa 800°C, 3 Stunden getempert (kalziniert). Das anschließende Abkühlen erfolgt äußerst langsam mit Abkühlgeschwindigkeiten von 1°C/min, um eine maximale Oxidation des Bismuts zu erreichen.

Die resultierenden katalytisch aktiven Barium-Bismutoxid-Verbindungen weisen eine Zusammensetzung der allgemeinen Formel $BaBi_yO_z$ auf, worin $y$ für einen Wert von 0,17 bis 3 und $z$ für einen Wert von 1,43 bis 5,6 steht. Deren Bariumgehalt liegt in einem Bereich von etwa 25 Schweratom-% (etwa 16 Gew. -%) bis etwa 90 Schweratom-%. Erfindungsgemäß wurde weiterhin die Bildung folgender neuer Phasen beobachtet:

a) $BaBi_3O_{5,6}$ - $BaBi_{1,33}O_{3,4}$
Phase mit pseudokubischer Perowskit-Struktur;
b) $BaBi_{1,32}O_{3,41}$ - $BaBi_{0,8}O_{3,04}$
Phase mit monokliner Perowskit-Überstruktur;
c) $BaBi_{0,79}O_{3,04}$ - $BaBi_{0,7}O_3$
Phase mit rhomboedrischer Perowskit-Überstruktur;
d) $BaBi_{0,69}O_3$ - $BaBi_{0,41}O_{1,95}$
Phase mit Elpasolit-Struktur;
e) $BaBi_{0,4}O_{1,84}$ - $BaBi_{0,33}O_{1,82}$
Phase mit tetragonaler Perowskit-Überstruktur;
f) $BaBi_{0,32}O_{1,82}$ - $BaBi_{0,28}O_{1,68}$
Phasengemisch des Bismutats mit tetragonaler Perowskit-Überstruktur und einer hexagonalen Phase;
g) $BaBi_{0,27}O_{1,68}$ - $BaBi_{0,17}O_{1,43}$
Phase mit hexagonaler Struktur.

Die genannten Phasen spalten oberhalb von etwa 550°C Sauerstoff reversibel ab. Gleichzeitig erfolgt eine vollständige Reduktion des enthaltenen Bi +5 zu Bi +3.

Die Vorteile der Barium-Bismutoxid-Katalysatoren liegen in der beschriebenen einfachen Herstellung aus preiswerten Edukten, die allgemein in technischem Reinheitsgrad eingesetzt werden können. Des weiteren sind die erhaltenen Katalysatoren nicht hygroskopisch und unlöslich in den meisten organischen Lösungsmitteln. Die Barium-reichen Katalysatoren sind unlöslich in $HNO_3$ und $H_2SO_4$. Vorteilhafterweise treten bei den beschriebenen Bismutoxid-Verbindungen keine Korrosionsprobleme auf. Aufgrund der hohen Dichte und der Möglichkeit zur Herstellung grobkörniger Produkte zeigen die erfindungsgemäßen Katalysatoren eine gute Handhabbarkeit und Transportierbarkeit.

Ein weiterer Gegenstand der Erfindung sind Verfahren zur Herstellung von Chlor durch Oxidation von Chlorwasserstoff unter Verwendung eines oder mehrerer der vorgenannten Katalysatoren.

Die erfindungsgemäßen Katalysatoren können hierzu als Vollkatalysatoren eingesetzt oder vor Verwendung mit einem Bindemittel versetzt oder auf einen inerten Träger aufgebracht werden. Als Trägermaterialien kommen die hierfür üblichen Stoffe, wie Oxide, insbesondere Silikate, Alumosilikate, Zeolithe, Aluminiumoxide, Zirkoniumoxid, Carbide, wie SiC, Nitride, wie $Si_3N_4$, nichtflüchtige Chloride, wie z.B. NaCl, KCl, $CaCl_2$ und $MgCl_2$, oder deren Mischungen, in Frage. Bevorzugte Träger sind $SiO_2$, SiC und $Si_3N_4$.

Die hohe Aktivität der Bismutoxid-Verbindungen bei der Oxidation von Chlorwasserstoff beruht auf deren Anteil von Bismut in der Oxidationsstufe +5, der zu einer sehr schnellen Chlorwasserstoffoxidation führt, wobei gleichzeitig Bi +5 zu Bi +3 reduziert wird. Würde nach vollständiger Reduktion des Bi +5 zu Bi+ 3 weiter Chlorwasserstoff zugeführt werden, bestünde die Gefahr einer unerwünschten Weiterreaktion zu den entsprechenden Bismut +3 oxichloriden, wie z.B. BiOCl, und Alkali- bzw. Erdalkalichloriden, wie z.B. $BaCl_2$. Da sich diese nicht mehr mit Sauerstoff in die erfindungsgemäßen Oxide umwandeln lassen, wäre der Katalysator dann nicht mehr regenerierbar. Durch geeignete Reaktionsführung muß daher bewirkt werden, daß die beschriebene Weiterreaktion vermieden wird. Dieses Problem wird gelöst, indem man den Katalysator nur teilreduziert, d.h. die Reaktion abbricht, bevor die mittlere Oxidationsstufe des Bismuts auf +3 gesunken ist. Durch geeignete Chlorwasserstoff- und/oder Sauerstoffdosierung und gegebenenfalls durch Wahl eines geeigneten Reaktortyps wird, wie im folgenden noch näher ausgeführt, ein lokaler Chlorwasserstoffüberschuß vermieden.

Die Oxidation von Chlorwasserstoff zur Chlorherstellung kann unter Verwendung der erfindungsgemäßen Katalysatoren in verschiedenen Verfahrensvarianten erfolgen, wobei man allgemein zwischen stationärer und instationärer Reaktionsführung unterscheidet. Der Katalysator ist für beide Verfahrensvarianten geeignet.

Bei der stationären Reaktionsführung wird das zugeführte Chlorwasserstoff-Feed zusammen mit einem sauerstoffhaltigen Gas und eventuell weiteren Verdünnungsgasen, wie z.B. Argon oder Stickstoff, zeitlich kontinuierlich mit dem Katalysatorbett in Kontakt gebracht, wobei Chlorwasserstoff teilweise zu Chlor und Wasser oxidiert wird, die zusammen mit nicht umgesetztem Chlorwasserstoff und Sauerstoff den Reaktor ebenfalls kontinuierlich verlassen. Bei stationärer Reaktionsführung kann der erfindungsgemäße Katalysator als Festbett, Wirbelschicht oder Wanderbett bzw. Fließbett eingesetzt werden. Da für die stark exotherme Chlorwasserstoffoxidation im Festbett allerdings Probleme mit der Wärmeabfuhr auftreten können, ist eine Umsetzung in Wirbelschicht oder Wanderbett bzw. Fließbett gegebenenfalls günstiger.

Bei der instationären Reaktionsführung wird das Verfahren in einen Cyclus aus zwei Teilschritten zerlegt, der sich fortlaufend wiederholt. Dabei sind Trennprozeß und Wärmeaustausch in den Reaktor integriert, was zu einer Einsparung von Aufarbeitungsschritten, Apparaten und Energie führt. Bei den instationär geführten Deacon-Verfahren nach dem Stand der Technik, wie sie in DE 4 004 454, US 4 959 202, WO 91/06505 (entsprechend US 51 54 911 bzw. EP 0 500 728 B1) und EP 0 474 763 beschrieben werden, fungiert der Katalysator als Chlorspeicher. Der Reaktionscylcus besteht aus einem Beladeschritt, in dem der Katalysator, der über eine oxidische Aktivkomponentenphase verfügt, mit Chlorwasserstoff beladen wird und einen anschließenden Regenerierungsschritt, bei dem ein sauerstoffhaltiges Gas über den Katalysator strömt, diesen regeneriert, wobei Chlor freigesetzt wird.

Im erfindungsgemäßen Verfahren mit instationärer Reaktionsführung fungiert der erfindungsgemäße Katalysator auf Bismutbasis als Sauerstoffspeicher. Im Chlorwasserstoff-Oxidationsschritt wird Chlorwasserstoff am Katalysator in Abwesenheit von freiem molekularen Sauerstoff zu Chlor und Wasser oxidiert, wobei der Katalysator Gittersauerstoff abgibt und teilreduziert wird. Im anschließenden Regenerierungsschritt strömt ein sauerstoffhaltiges Gas über den teilreduzierten Katalysator, der dabei reoxidiert wird. Bei diesem Verfahren wird bereits im ersten Schritt Chlorwasserstoff guantitativ zu Chlor und Wasser umgesetzt, so daß im Produktgas keine Salzsäure mehr enthalten ist und somit das Problem der Korrosion bei der anschließenden Aufarbeitung entschärft ist. Weitere Vorteile gegenüber den instationär geführten Deacon-Verfahren nach dem Stand der Technik ergeben sich daraus, daß das Chlor direkt aus Chlorwasserstoff gebildet und nicht zwischengespeichert wird. Dadurch werden die aus der Bildung flüchtiger Chloridintermediate der Katalysatoren resultierenden Probleme, wie Katalysatoraustrag und Aktivitätsabnahme und Verunreinigung der nachfolgenden Leitungsteile umgangen. Ebenso entfallen Probleme, die aufgrund der Adsorption von Chlorwasserstoff am Trägermaterial auftreten und zu einer Verunreinigung des Produktgases führen, da wie erwähnt Chlorwasserstoff bereits in der ersten Stufe des Verfahrens quantitativ umgesetzt wird.

Aufgrund der oben erwähnten sehr hohen Oxidationsaktivität des Katalysators, infolge der enthaltenen Anteile der Oxidationsstufe +5, läuft die Chlorwasserstoffoxidation auch bei tiefen Temperaturen mit hohen Reaktionsgeschwindigkeiten ab, so daß auch bei Raumtemperatur noch ein rascher Umsatz erfolgt. Verfahrenstechnisch ergeben sich somit überraschende Vereinfachungen, da die thermodynamischen Gleichgewichte schnell eingestellt werden, so daß die Anforderungen an Durchmischung und Verweilzeit (VWZ) sinken.

Die Aßfangstemperatur der Chlorwasserstoffoxidation liegt bei 0°C bis 700°C, bevorzugt in einem Bereich von 25°C bis 600°C. Während der Reaktion kann die Temperatur aufgrund von Wärmeeffekten etwas schwanken. Da die Chlorwasserstoffoxidation mit einer

Reaktionsenthalpie $\Delta/H_R$ (700 K) von -58,6 kJ/mol stark exotherm ist, muß auf ausreichende Wärmeabfuhr geachtet werden. Da die erfindungsgemäßen Katalysatoren, wie oben erwähnt, als Sauerstoffspeicher dienen und somit keine flüchtigen Chloride gebildet werden und sie zudem mechanisch und thermisch stabil sind, werden lokale Überhitzungen von bis zu etwa 150 K toleriert, ohne daß ein Elementaustrag erfolgt.

Die Chlorwasserstoffkonzentration im Zustrom liegt bei 1 bis 100% Chlorwasserstoffanteil, wobei hohe Konzentrationen vorteilhaft sind. Bevorzugt werden Chlorwasserstoffkonzentration von 10 bis 100% eingesetzt.

Zwischen dem Oxidations- und dem Regenerierungsschritt wird der Reaktor vorzugsweise mit einem Spülgas frei von Chlorwasserstoff gespült. Als Spülgase werden Stickstoff, Edelgase, Kohlendioxid oder deren Mischungen eingesetzt.

Die Regenerierung des teilreduzierten Katalysators erfolgt mit sauerstoffhaltigen Gasen, wobei bevorzugt Luft, sauerstoffangereicherte Luft, reiner Sauerstoff oder Distickstoffmonoxid eingesetzt werden.

Die verfahrenstechnische Umsetzung der drei Teilschritte Oxidation von Chlorwasserstoff zu Chlor, Spülung und Regenerierung des Katalysators, kann sowohl im Gleichstrom- als auch im Gegenstromverfahren erfolgen. Allgemein wird aus Gründen einer vorteilhaften Wärmeführung mit Strömungsumkehr zwischen Oxidation und Regenerierung gearbeitet. Die Gasflüsse können bei den einzelnen Teilschritten unterschiedlich sein. Auch der Einsatz von Strömungsgradienten oder -stufen bei der Oxidation und Regenerierung, um die Verweilzeit und die Stoffmengenkonzentration in der Gasphase zu regeln, ist möglich. Auch die Beimischung eines Trägergases in Form eines Gradienten oder in diskreten Stufen zur Implementierung einer Verweilzeitrampe ist möglich.

Die Chlorabtrennung aus dem Produktgas kann z.B. durch Kondensation, Absorption in Lösungsmitteln und/oder Druckwechselabsorption erfolgen.

Lokale Chlorwasserstoffüberkonzentrationen und damit verbunden die vollständige Reduktion des erfindungsgemäßen Katalysators müssen aus den oben beschriebenen Gründen vermieden werden. Bei stationärer Reaktionsführung ist es deshalb zweckmäßig, einen Festbett-, Wirbelschicht- oder Wanderbett-Reaktor anzuwenden und die Reaktion unter Sauerstoffüberschuß und bei einer Temperatur, bei der die Reoxidation ausreichend schnell verläuft, durchzuführen. Da die Wärmeabfuhr im Festbett jedoch problematisch ist, sind Wirbelschicht und Wanderbett bevorzugt. Wirbelschichtreaktoren sind besonders gut geeignet. Da die Feststoffphase und somit der Katalysator in der Wirbelschicht näherungsweise das Verweilzeitverhalten eines idealen Rührkessels aufweist, also sehr gut rückvermischt ist, kommt es zu einer annähernd gleichmäßigen Reduktion aller Katalysatorteilchen. Der mittlere Reduktionsgrad der Katalystorpartikel ist deshalb zeitlich und räumlich invariant. Eine Temperaturabsenkung bei stationärer Reaktionsführung bewirkt eine Gleichgewichtsverlagerung in Richtung hoher Umsätze. Allerdings erfordert die Regenerierung mit Sauerstoff eine erhöhte Temperatur zur Erzielung ausreichender Reoxidationsgeschwindigkeiten.

Bevorzugte Temperaturbereiche für einen stationären Prozess liegen bei etwa 30 bis etwa 700°C für die Oxidation und bei etwa 500 bis 800°C für die Regenerierung.

Für die technische Umsetzung des erfindungsgemäßen instationären Verfahrens sind zwei Varianten, entweder die räumliche oder die zeitliche Trennung der beiden Teilschritte Oxidation und Regenerierung, gegebenenfalls unterbrochen durch einen Spülvorgang als weiterer Verfahrensschritt, möglich.

Bei der räumlichen Trennung kommt ein Wanderbett bzw. Fließbett oder eine zirkulierende Wirbelschicht z.B. unter Verwendung eines Riser-Reaktors zur Anwendung, so daß die Katalysatorteilchen aus der Chlorwasserstoffoxidationszone, nach Abtrennung der gebildeten Reaktionsprodukte Chlor und Wasser, zu einem separaten Regenerierungsreaktor geführt werden, in dem die Reoxidation durchgeführt wird. Der regenerierte Katalysator wird dann in die Chlorwasserstoffoxidationszone zurückgeführt. Der Prozeß ist kontinuierlich und cyclisch, da der Katalysator fortwährend im Kreis gefördert wird. Der Katalysator ist dabei hohen mechanischen Beanspruchungen ausgesetzt und muß daher über eine ausreichende Härte verfügen. Diese Anforderung wird von den erfindungsgemäßen Katalysatoren erfüllt.

Die zeitliche Trennung der beiden Teilschritte läßt sich mit einem Festbett oder einer konventionellen Wirbelschicht realisieren, indem periodisch zwischen der Chlorwasserstoffzuführung und dem Regenerierungsschritt, eventuell nach einer Spülphase mit Inertgas umgeschaltet wird. Mehrere alternierend betriebene Festbett- bzw. Wirbelschichtreaktoren können zu einem Wärmeverbund gekoppelt werden.

Bei instationärer Fahrweise ist eine Festbettanordnung weniger erfolgversprechend, da der Reduktionsgrad des Katalysators nicht einheitlich ist, sondern mit der Rohrlänge variiert. Da die reaktoreingangsseitigen Katalysator-Zonen während der gesamten Zyklusdauer ununterbrochen mit HCl in Kontakt stehen, besteht hier, nach vollständigem Abreagieren der Bi+5-Zentren, die Gefahr unerwünschter Chlorierungsfolgereaktionen, noch bevor die HCl-Front die reaktorausgangsseitigen Zonen erreicht hat, deren Sauerstoffspeicher noch voll aufgeladen ist. Mehrfacheinspeisung von HCl und/oder Sauerstoff zur Einstellung möglichst homogener Konzentrationsverteilungen sind möglich, jedoch mit hohem Aufwand verbunden. Besonders bevorzugt wird daher die Fahrweise mit HCl-Unterschuß in der Wirbelschicht oder im Wanderbett.

In der Wirbelschicht wird die gesamte Katalysatormasse aufgrund der guten Durchmischung der Partikel gleichmäßig reduziert. Bei rechtzeitigem Abbruch der HCl-Oxidation und Umschalten auf Regenerierung kann die mittlere Bi-Oxidationsstu£e über +3 gehalten werden. Die obenbeschriebenen unerwünschten Folgereaktionen werden dadurch verhindert. Diese Ausführung des erfindungsgemaßen Verfahrens mit HCl-Unterschuß erfordert zur Erzielung hoher Raum-Zeit-Ausbeuten relativ große Katalysatormengen, die aber keinen entscheidenden Kostenfaktor für das erfindungsgemäße Verfahren darstellen, da die erfindungsgemäßen Katalysatoren kostengünstig herstellbar sind.

Eine andere bevorzugte Variante basiert auf der Verwendung eines Wanderbettes. Zirkulierende Wirbelschichten in der Ausgestaltung eines Riser- oder Dropper-Reaktors werden darin explizit eingeschlossen. Der Chlorwasserstoff-Zustrom (Feed) kann im Gleich- oder Gegenstrom zum Wanderbett geführt werden.

Im Riser werden beispielsweise die Katalysatorpartikel durch das HCl-Gas mitgerissen und in Strömungsrichtung transportiert. Die Verweilzeiten im Riser sind üblicherweise recht kurz und der Feststoffanteil recht hoch, so daß der Katalysator keineswegs tief durchreduziert wird, sondern der Nutzungsgrad eher gering ist. Daher ist der Sauerstoffspeicher immer gut gefüllt, so daß keine Gefahr einer irreversiblen Chlorierung besteht.

Im Dropper werden die Katalysatorpartikel unter Ausnutzung der Schwerkraft transportiert. Ansonsten ist die Funktionsweise des Droppers vergleichbar der des Risers.

Im folgenden wird die Funktionsweise des erfindungsgemäßen Verfahrens unter Verwendung eines Wanderbett-Reaktors erläutert. HCl-Gas strömt über eine Katalysator-Schüttung, so daß es zur Ausbildung eines Gradienten des Katalysator-Reduktionsgrades kommt. Am Anfang der Schüttung wird der Katalysator stärker reduziert als in den nachfolgenden Zonen. Am Ende der Schüttung wird dem Sauerstoffspeicher kaum noch Sauerstoff entnommen. Zur Erzielung von quantitativem Chlorwasserstoff-Umsatz ist eine Nachschüttung von noch nicht reduziertem Katalysator notwendig. Wenn nun der Oxidationsgrad der Anfangszone soweit gesunken ist, daß zur Verhinderung der Folgereaktion die Regenerierung notwendig ist, wird nur diese Zone, kontinuierlich oder in diskreten Schritten, aus der Reaktionszone entfernt, um dann extern reoxidiert zu werden. Am Ende der Schüttung wird mit frischem Katalysator aufgefüllt und die HCl-Oxidation fortgesetzt. Im erfindungsgemäßen Beispiel 1 wird gezeigt, daß durch diese Vorgehensweise die Chlorierung des Katalysators vollständig vermieden werden kann und der teilreduzierte Katalysator reversibel regenerierbar ist, d.h. nach Reoxidation die ursprüngliche Aktivität besitzt und denselben Bi+5-Gehalt wie zuvor aufweist.

Ein weiterer Gegenstand der Erfindung betrifft die Verwendung der vorgenannten Katalysatoren in heterogen katalysierten Oxidations- und Dehydrierreaktionen, insbesondere solchen Reaktionen, die in der Gasphase ablaufen. Beispiele für solche Reaktionen sind: die Oxidation von Halogenwasserstoffen zu den Halogenen, die oxidative Methankopplung, die Dehydrokopplung von substituierten Toluolen zu den entsprechenden Stilbenen, die oxidative Dehydrierung von Cycloalkanen zu Aromaten, die oxidative Dehydrierung von Vinylcyclohexan zu Ethylbenzol/Styrol und die Epoxidation von Olefinen.

Die Herstellung und Verwendung der erfindungsgemäßen Katalysatoren soll anhand der folgenden, nicht einschränkenden Beispiele näher erläutert werden.

Beispiel 1:

Herstellung eines Barium-Bismutoxid-Katalysators

5,42 g $Bi_2O_3$ und 5,67 g $BaCO_3$ werden in Aceton homogenisiert und in einem Kammerofen an der Luft bei 800°C kalziniert. Die Umsetzungsbedingungen werden wie folgt gewählt: Aufheizgeschwindigkeit 2°C/min., Haltezeit bei 800°C 3h, Abkühlgeschwindigkeit 1°C/min. Um die Homogenität des Präparats zu gewährleisten, wird dieses anschließend gemörsert und nochmals unter den vorgenannten Reaktionsbedingungen behandelt.

Der Katalysator wurde mittels Pulverröntgenbeugung charakterisiert. Die exakte Schweratom-Zusammensetzung wurde mittels Röntgenfluoreszenzspektroskopie und chemischer Analyse ermittelt. Der Gehalt an Bi+5 wurde durch iodometrische Titration und Differentialthermoanalyse (DTA/TG) ermittelt. Der Katalysator ist phasenrein und hat eine rhomboedrisch verzerrte Perowskit-Überstruktur. Er ist thermisch und mechanisch stabil und kann ohne Zerstörung der Phase bis ca. 900°C kalziniert werden. (Ab ca. 550°C erfolgt eine reversible $O_2$-Abspaltung). Gleichzeitig finden folgende reversible Phasenumwandlungen statt:

- 620°C -   Bildung einer Perowskit-Phase
- 750°C -   Umwandlung in eine Phase mit tetragonaler Perowskit-Struktur
- 820°C -   Phasenumwandlung in eine tetragonale Phase mit Perowskit-Überstruktur

Die letztgenannte Phase enthält kein Bi(5) mehr! Der Katalysator ist an Luft stabil, nicht flüchtig, unlöslich in Laugen, jedoch löslich in wäßrigen Säuren.

Der Katalysator hat die Zusammensetzung $BaBi_{0,81}O_{2,6}$. Der Bi+5-Gehalt liegt bei 47,85% (bezogen auf Gesamtbismut-Gehalt) und wurde durch iodometrische Titration nach Bunsen ermittelt.

Die Farbe des oxidierten Katalysators ist dunkelbraun. Die reduzierte Phase ist dunkelrot. Das bei der Festkörperreaktion anfallende Präparat besitzt eine poröse Struktur.

Beispiel 2:

Herstellung von Chlor aus gasförmiger trockener verdünnter HCl im Wanderbettreaktor unter instationärer Reaktionsführung

In ein wassergekühltes Reaktionsrohr werden 10 g des Katalysators in vier gleich großen Portionen gefüllt. Als Absperrung zwischen den Schichten wird Glaswolle verwendet. Der Katalysator wird durch Wasserkühlung auf Raumtemperatur abgekühlt. Mittels Kolbenprober wird 100 ml HCl, welches mit 300 ml Argon verdünnt wird, eingespritzt. Das bei der Reaktion entstandene Chlor wird im schwachen Argonstrom in eine Vorlage mit eisgekühlter KI-Lösung geleitet. Das dabei gebildete $I_2$ wurde mit 0,1 n Thiosulfat-Lösung und Stärke als Indikator titriert.

Aus dem Einsatz von $4,4 \times 10^{-3}$ mol HCl-Gas errechnet sich ein theoretischer Verbrauch von 43,82 ml Thiosulfat-Lösung entsprechend $2,2 \times 10^{-3}$ mol $Cl_2$. Der tatsächliche Verbrauch betrug 43,52 ml Thiosulfat-Lösung entsprechend $2,18 \times 10^{-3}$ mol $Cl_2$. Damit erfolgte eine annähernd vollständige (99,3%) Umsetzung von HCl.

Proben der mit HCl reagierten Katalysatorabschnitte 1 und 2 wurden auf eventuell gebildete Chloride mittels $AgNO_3$-Lösung untersucht. Der Nachweis fiel negativ aus. Nach weiterer Reaktion mit 100 ml verdünnter HCl (99,5%ige Umsetzung) wurde die gesamte Katalysatormasse auf Chloride untersucht. Dabei konnten erneut keine Chloride nachgewiesen werden.

Die Katalysatormasse wurde zur Regenerierung an der Luft bei 550°C im Kammerofen unter folgenden Reaktionsbedingungen kalziniert: Aufheizgeschwindigkeit 10°C/min. Haltezeit bei 550°C 2 Stunden. Abkühlgeschwindigkeit ca. 3°C/min. Eine anschließend durchgeführte iodometrische Titration zeigte die vollständige Regeneration des Katalysators.

Entsprechend den obigen Bedingungen wurde die Umsetzung mit $2 \times 100$ ml verdünnter HCl ein weiteres Mal wiederholt. Die Umsetzung lief dabei mit 99,4- bzw. 99,6%iger Ausbeute ab.

Wie der Versuch zeigt, kann durch Wahl eines Wanderbettes und Verdünnung der HCl ein vollständiger Umsatz HCl zu Chlor erreicht werden (die Bestimmung der gebildeten Chlor-Menge durch iodometrische Titration erfolgt mit einem Fehler von ca. ± 0,5%). Der Katalysator ist vollständig regenerierbar. Die Bildung von Ba- und Bi-Chloriden bei HCl-Unterschuß konnte nicht beobachtet werden, d.h. die irreversible Chlorierung des Katalysators kann durch ein Wanderbett wirkungsvoll verhindert werden.

Wichtig ist dabei eine rechtzeitige Entfernung des gebildeten teilreduzierten Ba-Bismutatoxid-Katalysators aus dem Reaktionsraum, da sonst eine weitere Reaktion mit HCl stattfindet. Vorzugsweise werden die einzelnen Wanderbettzonen bereits aus dem Reaktor abgezogen und regeneriert, noch bevor die mittlere Bi-Oxidationsstufe auf +3 gesunken ist.

Eine Kühlung des Katalysators auf Raumtemperatur wie durch Einsatz eines wassergekühlten Reaktorrohres erreicht, ist nicht zwingend notwendig. Sie diente nur zur Demonstration der hohen Katalysatoraktivität.

Vergleichsbeispiel 1:

Herstellung von Chlor aus wäßriger HCl-Lösung im Rührkesselreaktor unter instationärer Reaktionsführung

In einem 250 ml Dreihalskolben werden 10 g des Katalysators aus Beispiel 1 eingewogen. Im Stickstoffstrom werden 3,5 ml konzentrierte wäßrige HCl (37%) zugetropft. Das bei der Umsetzung gebildete Chlor wird im Stickstoffstrom durch einen wassergekühlten Rückflußkühler in eine Vorlage mit 500 ml eisgekühlter KI-Lösung geleitet. Das dabei gebildete $I_2$ wird mit 0,1 n Natriumthiosulfatlösung und Stärke als Indikator titriert.

Theoretischer Verbrauch: ca. 220 ml Thiosulfat-Lösung entsprechend 2,3 g Bi(+5) ($1,1 \times 10^{-1}$ mol) bzw. 0,39 g $Cl_2$.

Tatsächlicher Verbrauch 157 ml Thiosulfat-Lösung entsprechend 1,64 g Bi+5.

Der Versuch zeigt, daß der Katalysator so hochaktiv ist, daß die HCl-Oxidation zu Chlor unter Reduktion des Katalysators sogar schon bei Raumtemperatur stattfindet. Dennoch ist unter diesen Bedingungen (wäßrige HCl) und bei dieser Art der Reaktionsführung (Rührkessel) keine technische Realisierung des erfindungsgemäßen Verfahrens möglich, da der Katalysator ebenfalls chloriert wird, d.h. es bilden sich Chloride selbst bei HCl-Unterschuß.

Vergleichsbeispiel 2:

Herstellung von Chlor aus gasförmigem trockenen HCl-Gas unter instationärer Reaktionsführung

In ein Reaktionsrohr werden 10 g des Katalysators aus Beispiel 1 gefüllt. Mittels Kolbenprober wird 200 ml HCl, welche mit 200 ml Ar verdünnt wird, eingespritzt. Das bei der Reaktion entstandene Chlor wird im schwachen Argonstrom in eine Vorlage mit eisgekühlter KI-Lösung geleitet. Das dabei gebildete $I_2$ wird mit 0,1 n Thiosulfat-Lösung und Stärke als Indikator titriert.

Aus dem Einsatz von $8,8 \times 10^{-3}$ mol HCl-Gas errechnet sich ein theoretischer Verbrauch von 87,64 ml Thiosulfat-Lösung entsprechend $4,4 \times 10^{-3}$ mol $Cl_2$. Der tatsächliche Verbrauch betrug 52,43 ml Thiosulfat-Lösung entsprechend $2,63 \times 10^{-3}$ mol $Cl_2$. Somit erfolgte lediglich eine 59,8%ige Umsetzung.

Die mit der HCl reagierten Katalysatorabschnitte 1 und 2 wurden auf Chloride mittels $AgNO_3$-Lösung untersucht. Dabei konnte $Cl^-$ nachgewiesen werden.

Das bei der Oxidation der HCl gebildete Ba-Bismut(+3)oxid reagiert mit HCl in einer 2. Stufe weiter. Im vorderen Bereich der Katalysatormasse kann die Bil-

dung von BiOCl und BaCl$_2$ nachgewiesen werden. Der Katalysator wird wie in Vergleichsbeispiel 1 irreversibel chloriert.

**Beispiel 3:**

Abhängigkeit der Kristallstruktur von der Stöchiometrie der Schweratome Barium und Bismut

Unter Anwendung der in Beispiel 1 beschriebenen Verfahrensbedingungen und Variation der Einsatzmengen von Bi$_2$O$_3$ und BaCO$_3$ sind die oben erwähnten Bismutoxid-Phasen a) bis g) herstellbar.

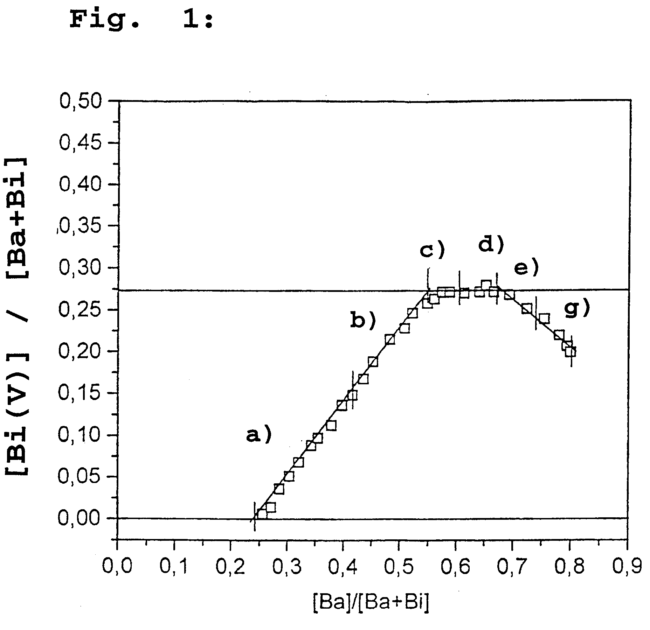

Fig. 1 zeigt für die einzelnen Phasen das Verhältnis vom Bi+5-Gehalt bezogen auf den Gesamtschweratom-Gehalt (y-Achse) zum Ba-Gehalt bezogen auf den Gesamtschweratomgehalt. Die eingezeichnete waagerecht Gerade kennzeichnet den maximalen Bi+5-Gehalt (27,5 Schweratom-%).

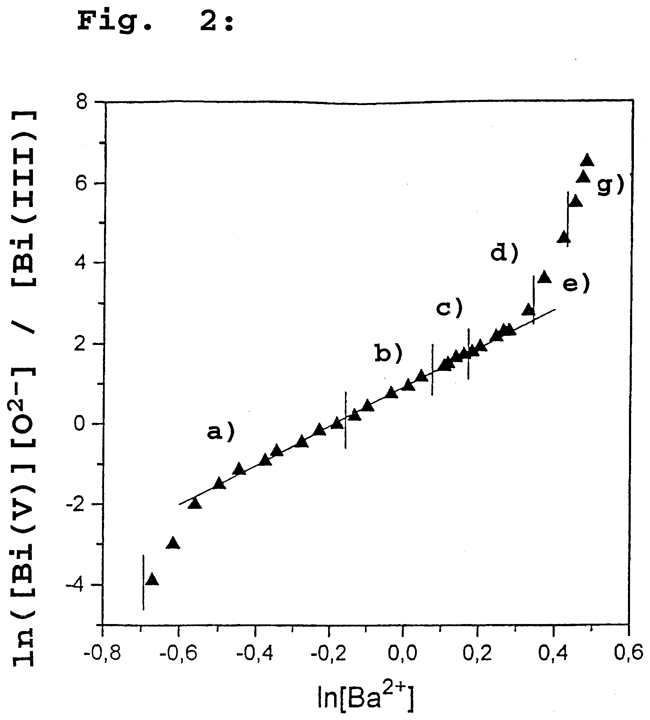

Fig. 2 zeigt für die einzelnen Phasen in doppelt logarithmischer Auftragung die Beziehung des Bi +5 / Bi +3-Redoxsystems (y-Achse) zum Bariumgehalt (x-Achse).

Die eingezeichnete Gerade, entsprechend einem Bariumgehalt von etwa 30 bis etwa 65 Schweratom-%, kennzeichnet einen Bereich, in dem die Bi +5-Konzentration in Abhängigkeit von der Ba-Konzentration nach folgender Gleichung:

$$\ln \frac{[Bi\,(V)]\,[O^{2-}]}{[Bi\,(III)]} - 4{,}86(4) - \ln [Ba^{2+}] = K$$

steigt. Diese Beziehung steht in Analogie zum Massenwirkungsgesetz für die Redoxreaktion zwischen Bi +3 und Bi +5:

$$Bi\,(III) + 1/2\,O_2 \leftrightharpoons Bi\,(V) + O^{2-}$$

Im linearen Bereich der doppelt logarithmischen Auftragung, gekennzeichnet durch die eingezeichnete Gerade, verhalten sich die vier verschiedenen, auf ihr liegenden Phasen a, b, c und d wie eine einzige homogene Phase.

Die Bestimmung des Bi +5-Gehalts erfolgt durch jodometrische Titration nach Bunsen.

**Patentansprüche**

1. Katalysatorzusammensetzung, dadurch gekennzeichnet, daß deren Aktivphase wenigstens eine Bismutoxidverbindung enthält, worin Bismut wenigstens teilweise in der Oxidationsstufe +5 vorliegt, wobei die Bismutoxidverbindung außerdem wenigstens eine die Oxidationsstufe +5 stabilisierende, basische Metallkomponente enthält.

2. Katalysatorzusammensetzung nach Anspruch 1, dadurch gekennzeichnet, daß der Anteil an Bi$^{+5}$, bezogen auf den Gesamtbismutgehalt, etwa 1 bis 99 Gew.-%, bevorzugt 5 bis 90 Gew.-%, insbesondere 20 bis 80 Gew.-% beträgt.

3. Katalysatorzusammensetzung nach Anspruch 1, dadurch gekennzeichnet, daß die basische Metallkomponente ausgewählt ist unter wenigstens einem Alkali-, Erdalkali- und Seltenerdmetall.

4. Katalysatorzusammensetzung nach Anspruch 3, dadurch gekennzeichnet, daß die basische Metallkomponente ausgewählt ist unter Li, Na, K, Sr, Ba, Cs, Y und La.

5. Katalysatorzusammensetzung nach einem der vorhergehenden Ansprüche, dadurch gekennzeichnet, daß die Aktivphase mindestens eine Verbindung der allgemeinen Formel

$$KBi_yO_z$$

worin

y    für einen Wert von 0,5 bis 2 und

z    für einen Wert von 1,25 bis 5,5 steht,

oder der allgemeinen Formel:

$$BaBi_yO_z$$

worin

y    für einen Wert von 0,17 bis 3 und

z    für einen Wert von 1,43 bis 5,6 steht,

enthält,
wobei Bi jeweils wenigstens teilweise in der Oxidationsstufe +5 vorliegt.

6. Katalysatorzusammensetzung Anspruch 5, dadurch gekennzeichnet, daß der Quotient y/z in Verbindungen der allgemeinen Formel

$$KBi_yO_z,$$

im Bereich von etwa 0,4 bis etwa 0,36 liegt; und in Verbindungen der allgemeinen Formel

$$BaBi_yO_z$$

im Bereich von etwa 0,12 bis etwa 0,54 liegt.

7. Katalysatorzusammensetzung nach einem der vorhergehenden Ansprüche, dadurch gekennzeichnet, daß die Aktivphase außerdem wenigstens einen Promotor, ausgewählt unter Elementen der 1., 2. und 4. Hauptgruppe sowie der 4. und/oder 6.

Nebengruppe des Periodensystems, enthält.

8. Katalysatorzusammensetzung nach einem der vorhergehenden Ansprüche, und zwar

    a) in Form eines Vollkatalysators;
    b) versetzt mit einem Bindemittel; oder
    c) aufgebracht auf einem inerten Träger.

9. Verbindung der allgemeinen Formel

$$BaBi_yO_z$$

worin

y    für einen Wert von etwa 0,17 bis 3 und
z    für einen Wert von etwa 1,43 bis 5,6 steht,

ausgenommen Verbindungen, worin

    y für einen Wert von 0,667 und z für einen Wert von 2,487,
    y für einen Wert von 0,600 und z für einen Wert von 2,378,
    y für einen Wert von 0,538 und z für einen Wert von 2,285,
    y für einen Wert von 0,504 und z für einen Wert von 2,218,
    y für einen Wert von 0,429 und z für einen Wert von 2,057,
    y für einen Wert von 0,333 und z für einen Wert von 1,835

steht,
wobei Bi jeweils wenigstens teilweise in der Oxidationsstufe +5 vorliegt.

10. Verbindung nach Anspruch 9, wobei der Quotient y/z im Bereich von etwa 0,12 bis 0,54 liegt.

11. Verbindung nach einem der Ansprüche 9 oder 10, gekennzeichnet durch eine der folgenden Zusammensetzungen und Kristallstrukturen:

    a) $BaBi_3O_{5,6}$ - $BaBi_{1,33}O_{3,4}$
    mit pseudokubischer Perowskit-Struktur;
    b) $BaBi_{1,32}O_{3,41}$ - $BaBi_{0,8}O_{3,04}$
    mit monokliner Perowskit-Überstruktur;
    c) $BaBi_{0,79}O_{3,04}$ - $BaBi_{0,7}O_3$
    mit rhomboedrischer Perowskit-Überstruktur;
    d) $BaBi_{0,69}O_3$ - $BaBi_{0,41}O_{1,95}$
    mit Elpasolit-Struktur;
    e) $BaBi_{0,4}O_{1,84}$ - $BaBi_{0,33}O_{1,82}$
    mit tetragonaler Perowskit-Überstruktur;
    f) $BaBi_{0,32}O_{1,82}$ - $BaBi_{0,28}O_{1,68}$
    Phasengemisch des Bismutats mit tetragonaler Perowskit-Überstruktur und einer hexagonalen Phase;

    g) $BaBi_{0,27}O_{1,68}$ - $BaBi_{0,17}O_{1,43}$
    mit hexagonaler Struktur.

12. Verfahren zur Herstellung einer Verbindung nach einem der Ansprüche 9 bis 11, dadurch gekennzeichnet, daß eine oder mehrere Bariumverbindungen, ausgewählt unter Bariumnitrat, -oxid, -peroxid, -hydroxid und -carbonat, mit einer oder mehreren Bismutverbindungen, ausgewählt unter Bismutoxid, -nitrat, -carbonat oder -hydroxid, umgesetzt werden und die Umsetzung naßchemisch oder als Festkörperreaktion erfolgt.

13. Verfahren nach Anspruch 12, dadurch gekennzeichnet, daß man $BaCO_3$ und/oder $BaO_2$ mit $Bi_2O_3$ in stöchiometrischen Mengen vermischt und das Gemisch bei einer Temperatur im Bereich von etwa 700°C bis etwa 900°C kalziniert.

14. Verfahren zur Herstellung von Chlor durch Oxidation von Chlorwasserstoff, dadurch gekennzeichnet, daß man einen Katalysator nach einem der Ansprüche 1 bis 8 mit Chlorwasserstoff in Kontakt bringt und den Katalysator reoxidiert, bevor die mittlere Oxidationsstufe von Bismut auf +3 sinkt.

15. Verfahren nach Anspruch 14, gekennzeichnet durch eine stationäre Verfahrensführung, wobei man Chlorwasserstoff zusammen mit einem sauerstoffhaltigen Gas mit dem Katalysator in Kontakt bringt und Chlorwasserstoff (teilweise) zu Chlor oxidiert.

16. Verfahren nach Anspruch 14 oder 15, dadurch gekennzeichnet, daß der Katalysator als Festbett, Wirbelschicht, Wanderbett oder Fließbett zur Anwendung kommt.

17. Verfahren nach Anspruch 14, gekennzeichnet durch eine instationäre Verfahrensführung, wobei man

    a) den Katalysator mit einem Chlorwasserstoffhaltigen, sauerstofffreien Gas in Kontakt bringt, Chlorwasserstoff zu Chlor oxidiert und die Reaktionsprodukte entfernt, und
    b) wenigstens einen Teil der gesamten Katalysatormasse in Gegenwart eines sauerstoffhaltigen Gases reoxidiert.

18. Verfahren nach Anspruch 17, dadurch gekennzeichnet, daß der Katalysator als Wirbelschicht, Wanderbett oder Fließbett zur Anwendung kommt.

19. Verwendung eines Katalysators nach einem der Ansprüche 1 bis 8 oder einer Verbindung nach einem der Ansprüche 9 bis 11 in heterogenkatalytischen Oxidations- oder Dehydrierreaktionen.

20. Verwendung nach Anspruch 19, nämlich zur Oxidation von Halogenwasserstoffen zu den Halogenen, oxidativen Methankopplung, Dehydrokopplung von substituierten Toluolen zu den entsprechenden Stilbenen, oxidativen Dehydrierung von Cycloalkanen zu Aromaten, oxidativen Dehydrierung von Vinylcyclohexen zu Ethylbenzol/Styrol und Epoxidation von Olefinen.

21. Verwendung nach Anspruch 20, nämlich zur Oxidation von Chlorwasserstoff zu Chlor.

**Fig. 1:**

Fig. 2:

**EP 0 826 417 A1**

Europäisches Patentamt

**EUROPÄISCHER RECHERCHENBERICHT**

Nummer der Anmeldung

EP 97 11 4611

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A,D | WO 91 06505 A (UNIV SOUTHERN CALIFORNIA) * das ganze Dokument * --- | 1-21 | B01J23/18 C01B7/04 C07F9/94 |
| P,A | EP 0 761 594 A (BASF AG) * das ganze Dokument * * Seite 2, Spalte 23, Zeile 39 * --- | 1-21 | |
| A | GB 2 297 043 A (BASF AG) * das ganze Dokument * ----- | 1-21 | |

**RECHERCHIERTE SACHGEBIETE (Int.Cl.6)**

B01J
C01B
C07F

Der vorliegende Recherchenbericht wurde für alle Patentansprüche erstellt

| Recherchenort | Abschlußdatum der Recherche | Prüfer |
|---|---|---|
| DEN HAAG | 10.Dezember 1997 | Dack, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................
& : Mitglied der gleichen Patentfamilie, übereinstimmendes Dokument

EPO FORM 1503 03.82 (P04C03)

14